(19) Europäisches Patentamt / European Patent Office / Office européen des brevets

(11) **EP 3 143 984 B1**

(12) **EUROPEAN PATENT SPECIFICATION**

(45) Date of publication and mention
of the grant of the patent:
**04.09.2019 Bulletin 2019/36**

(21) Application number: **15793463.9**

(22) Date of filing: **23.04.2015**

(51) Int Cl.:
**A61K 8/891** (2006.01)    **A61K 8/02** (2006.01)
**A61K 8/31** (2006.01)    **A61K 8/34** (2006.01)
**A61K 8/37** (2006.01)    **A61K 8/81** (2006.01)
**A61K 8/894** (2006.01)    **A61K 8/895** (2006.01)
**A61K 8/92** (2006.01)    **A61Q 1/00** (2006.01)
**A61Q 19/00** (2006.01)    **A61K 8/58** (2006.01)
**A61K 8/06** (2006.01)    **A61Q 19/02** (2006.01)

(86) International application number:
**PCT/JP2015/062403**

(87) International publication number:
**WO 2015/174241 (19.11.2015 Gazette 2015/46)**

(54) **STICK-SHAPED COSMETIC FOR MOISTURE REPLENISHMENT**

STIFTFÖRMIGE KOSMETIKA ZUR FEUCHTIGKEITSNACHFÜLLUNG

COSMÉTIQUE SOUS FORME DE BÂTON POUR HYDRATER LA PEAU

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **14.05.2014 JP 2014100358**

(43) Date of publication of application:
**22.03.2017 Bulletin 2017/12**

(73) Proprietor: **Shiseido Company, Ltd.
Chuo-ku
Tokyo 104-0061 (JP)**

(72) Inventors:
• **MATSUFUJI, Yoko
Yokohama-shi
Kanagawa 224-8558 (JP)**
• **WATANABE, Hiroko
Yokohama-shi
Kanagawa 224-8558 (JP)**
• **NOMIZU, Chieko
Yokohama-shi
Kanagawa 224-8558 (JP)**

• **TSUNENAGA, Makoto
Yokohama-shi
Kanagawa 224-8558 (JP)**
• **OOTAKE, Sawako
Yokohama-shi
Kanagawa 224-8558 (JP)**
• **FURUKAWARA, Tomomi
Yokohama-shi
Kanagawa 224-8558 (JP)**

(74) Representative: **Ter Meer Steinmeister & Partner
Patentanwälte mbB
Nymphenburger Straße 4
80335 München (DE)**

(56) References cited:
**EP-A2- 1 036 553**    **WO-A1-98/18438**
**JP-A- 2002 053 425**    **JP-A- 2006 028 179**
**JP-A- 2010 535 856**    **JP-A- 2013 010 744**
**JP-A- 2013 227 295**

**Description**

Technical Field

The present invention relates to a stick cosmetic (a cosmetic in stick-shape) used for replenishing skin moisture. More particularly, the present invention relates to a stick cosmetic that has a function to replenish skin moisture and moreover has superior shape-stability of the stick. The stick cosmetic is convenient because of its portability. The stick cosmetic according to the present invention can be suitably used for fixing (touching up) an impaired makeup such as spoiling quality of foundation, and is particularly suited, combined with its portability, for fixing cosmetic such as re-makeup when being out of doors (outdoor).

Background Art

A keeping moistening the skin and providing a barrier capable of protecting the skin from a variety of stimuli are basics for the skin-care. On the other hand, phenomena in which the dried skin due to a persistent dryness in winter and drying-effect on the skin due to air-conditioning in summer-time and the like take place inevitably.

It is known that a mist cosmetic is used as a means for replenishing dry skin with moisture. The mist cosmetic sprayed on the dry skin is capable of readily replenishing moisture on the skin. However, the mist cosmetic should contain a sprayable liquid cosmetic and require a container to retain the liquid cosmetic, so that the portability thereof may be sacrificed and become poor. In addition, some mist cosmetics are housed in a small portable container, but a blendable amount of the oil, particularly the solid oil should be limited in order to avoid a defected suction as for a dispenser due to clogging with the mist per se or a viscosity increase thereof, and as a result, the persistence of the moisturizing effect is quite limited. In addition, other problems are that the mist cosmetic is, due to its liquidity, likely to drip off from the skin when applied, and that the mist cosmetic is repelled particularly from an oily foundation, so that an enough amount of the mist cosmetic cannot be sufficiently applied and satisfactory moisturizing effect cannot be thus obtained.

In contrast, a stick cosmetic has a solid form, and therefore can be miniaturized to become convenient for carrying. Currently, the most popular stick cosmetic applied to the skin is a stick foundation.

It is required that the stick cosmetic should be smoothly spreadable when applied to the skin with a suitable hardness so that the stick cosmetic can be directly applied to the skin (without using fingers). Specifically; it is problematic that when applied, if the hardness thereof is too low, the form thereof is deformed, and if the hardness thereof is too high, the application may be too hard to use due to the poor spreadability thereof. In addition, the time-course stability, i.e., the composition and the form thereof should not be changed for a long time, is mandatory therefor.

Patent Document 1 discloses that a hydrocarbon-based wax conventionally widely used as a solidified oil content required for the solidification of a water-in-oil stick cosmetic provided poorly "skin touch softness," and following such results, hydrogenated jojoba oil is mixed in a stick cosmetic to provide with a soft feel when applied. Patent Document 2 discloses that a stick cosmetic having ascorbic acid glucoside, also known as a skin-whitening agent, can be stably formed with further an excellent storage stability, i.e., the form is retained for a long time, and an excellent feeling of use. It is presumed that particularly a predetermined amount of a blended hydrocarbon wax including polyethylene wax contributes to improve the feeling of use and storage stability.

In addition, stick cosmetics to which a variety of functions are added also have been developed. For example, Patent Document 3 discloses an emulsified stick cosmetic of which stability and the strength do not worsened even if a large amount of an ultraviolet scattering agent is blended, and Patent Document 4 discloses a non-aqueous stick cosmetic having a makeup-cleansing function.

However, a water-in-oil emulsified stick cosmetic having a skin moisture replenishing function, as a mist cosmetic could have, and being capable of applying to fix a makeup has been unknown to date.

Prior Art Documents

Patent Documents

Patent Document 1: JP-A 2002-53425
Patent Document 2: JP-A 2010-285349
Patent Document 3: JP-A 2013-139403
Patent Document 4: JP-A 2003-342163

Summary of Invention

Problems to be solved by the Invention

Therefore, it is an object of the present invention to provide a stick cosmetic having the new functional effect on moisture replenishing during the daytime and can be used for fixing a makeup, despite a stick form. Means for solving the Problems

The present inventors have studied diligently in order to solve the above problem, and as a result, found that a stick cosmetic capable of replenishing skin moisture by application is obtained by blending a fluid volatile oil and a wax capable of gelatin the fluid volatile oil, further blending a silicone elastomer and a silicone-based surfactant, and also blending a larger amount of water than in a usual stick cosmetic to thereby provide a water-in-oil emulsion, and completed the present invention.

Specifically, the present invention provides a stick cosmetic for moisture replenishing as defined in claim 1.

A cosmetic of the present invention comprises a high water content despite a stick water-in-oil emulsified cosmetic. Particularly, the cosmetic of the present invention is directly applied to the skin because of a stick form thereof, so that a very dewy, pleasant, refreshing feeling and cold feeling can be given because the emulsion state is broken due to friction when applied and the water in the internal phase is sprung. Generally, it is required to blend a cooling component such as ethanol or menthol in order to provide a cool feeling on a cosmetic use, but the cosmetic according to the present invention can provide a preferable cool feeling without such a cooling component, and in addition, problems such as a unpleasant smell and a stimulus originated from the cooling component would not take place. In addition, the volatile liquid oil is blended, so that an oily filmy sense (oiliness) cannot be left on the skin. Further, the blended silicone elastomer helps to suppress the stick becoming "slim' and the form stability thereof is superior.

The cosmetic of the present invention has the properties as described above while having a stick shape excellent in portability and therefore is suited for fixing makeup, by which makeup imperfection is easily fixed even outside the house.

Embodiments for carrying out the Invention

A "moisture replenishing stick cosmetic" (hereinafter also simply referred to as a "cosmetic") according to the present invention will be described in detail below.

The cosmetic of the present invention comprises (A) a fluid volatile oil.

The "fluid volatile oil" in the present specification is defined as an oil that is fluid at normal temperature (25°C) and consisting of volatile hydrocarbon oils (the boiling point thereof is not higher than 260°C at normal pressure), silicone oils (for example, cyclic silicone oils) and less viscos linear or branched silicone oils (10 cs or less, preferably 5 cs or less). Such fluid volatile oils can be a single kind of oil or a mixture of more than two fluid volatile oils.

As the volatile hydrocarbon oil, low boiling point isoparaffin-based hydrocarbon oils are preferred. Specific examples can include isododecane and isohexadecane.

The volatile or low viscosity silicone oil includes cyclic dimethylpolysiloxane having 4 to 6 silicon atoms and chain dimethylpolysiloxane having 2 to 5 silicon atoms. Specific examples can include cyclic silicone oils such as hexamethylcyclotrisiloxane (D3), octamethyltetracyclosiloxane (D4), decamethylcyclopentasiloxane (D5), and dodecamethylcyclohexasiloxane (D6), diphenyl siloxyphenyl trimethicone, and dimethicone (10 cs, 6 cs, 5 cs, 2 cs, or 1.5 cs).

The amount of the fluid volatile oil (A) blended in the cosmetic of the present invention is 5 to 20% by mass. When the amount blended is less than 0.01% by mass, stickiness, glare, and the unevenness of the foundation occur. When more than 50% by mass of the fluid volatile oil (A) is blended, smooth spread is lost, and further the chipping and crumbling of the stick are likely to occur. In addition, dewiness at the time of application is lost.

The cosmetic of the present invention is a water-in-oil type emulsified cosmetic containing (B) water in the internal phase.

The above Patent Document 1 states that in the water-in-oil emulsified stick cosmetic described in the above Patent Document 1, the amount of water blended is preferably 25% by mass or less because as the amount of water blended becomes larger, the spread becomes heavier (see paragraph [0024]). But, on the contrary, in the composition of the cosmetic of the present invention, as the amount of water blended becomes smaller, a feeling of a film, greasiness, and stickiness due to the oil tend to appear, and also dewiness and a refreshing feeling (cold feeling) specific to the present invention tend to be lost. Therefore, the amount of water blended in the cosmetic of the present invention is 45% by mass or more. The upper limit value of the amount of water blended is not particularly limited and can be, for example, 80% by mass or less.

Because the cosmetic of the present invention comprises a large amount of water as described above, the emulsification breaks on the skin at the time of application to overflow the water in the internal phase, and thus a very dewy and fresh feel (or a cold feeling) can be given.

The cosmetic of the present invention is a stick cosmetic containing (C) a wax, and the wax is a wax capable of gelating the above fluid volatile oil (A).

In the present invention, "a wax capable of gelating a fluid volatile oil" means a wax such that an emulsion comprising a uniform oil gel formed by mixing 85% by mass of a fluid volatile oil (for example, decamethylcyclopentasiloxane) and 15% by mass of the wax to be blended into a cosmetic has a hardness of 5 or more, preferably 10 or more, and more preferably 15 or more. The upper limit of the hardness is 120 or less, preferably 100 or less, more preferably 50 or less, and further preferably 35 or less.

Here, the "hardness" in the above definition is hardness ($\gamma$) represented by the following formula (1) as measured under the following conditions using a known measuring apparatus such as a rheometer (manufactured by SUN) :

$$\gamma = (G*L)/(l*a)(N/cm^2) \ (1)$$

wherein, G: measured stress, L: the thickness (mm) of a sample, 1: compression distance (mm), a: the cross-sectional area (cm$^2$) of a needle.

(Measurement Conditions)

MODE: 20
R/H: Hold
P/T: Press
REP: 1
MAX: 2 kg
Penetration: 10.0 mm
Penetration rate: 300 mm/min.
Needle diameter: 1.5 mm $\varphi$
Measurement temperature: 25°C

Examples of the wax satisfying the above condition include waxes such as microcrystalline waxes, paraffin waxes, myristyl myristate, ceresin wax, candelilla wax, polyethylene wax, jojoba oil (hydrogenated jojoba oil), and rice wax, or solid hydrocarbons, or mixtures thereof.

The amount of the wax(C) blended in the cosmetic of the present invention is not limited as long as it is an amount sufficient to gelate the above fluid volatile oil to the above predetermined hardness. The amount blended is 3 to 10% by mass. When the amount blended is less than 0.1% by mass, the hardness may be too low, and therefore the shape of the stick may not be able to be kept. When more than 20% by mass of the (C) wax is blended, the hardness may be too high, and therefore the spread may be poor to cause a difficulty in application to the skin.

The cosmetic of the present invention further contains (D) a silicone elastomer. By blending the silicone elastomer, "thinning," which means that the stick becomes thin due to the evaporation of the volatile oil over time, can be suppressed to achieve excellent shape stability, and at the same time, a skin roughness (imperfection) correcting effect is also provided. Further, in molding into a stick shape in the production process, slow cooling is necessary in order to suppress a depression in the center, and the blending of the silicone elastomer is effective in suppressing the increase of emulsified particles before solidification by slow cooling.

The silicone elastomer used in the present invention include one or two or more powders of crosslinked silicone resins such as (dimethicone/phenyl vinyl dimethicone) crosspolymers, (dimethicone/vinyl dimethicone/methicone) crosspolymers, (dimethicone/vinyl dimethicone) crosspolymers, dimethicone crosspolymers, and (diphenyl dimethicone/vinyl diphenyl dimethicone/silsesquioxane) crosspolymers.

The silicone elastomer may be blended in a state of a silicone gel comprising a crosslinked silicone resin powder and a solvent. For such a silicone gel, commercial products can be used. Examples thereof can include KSG-18A, KSG-16, and KSG-15AP (manufactured by Shin-Etsu Chemical Co., Ltd.) and an elastomer blend DC9045 (manufactured by Dow Corning Toray Co., Ltd.). The fluid volatile oils contained in these silicone gels as solvents are classified into the above component (A), and the amount of the fluid volatile oil blended is added to the amount of the component (A) blended.

The amount of the silicone elastomer (D) blended in the cosmetic of the present invention is 0.1 to 2% by mass. When the amount blended is less than 0.005% by mass, a decrease in emulsification stability, the thinning of the stick, and a decrease in the roughness correcting effect when fixing makeup may occur. When more than 10% by mass of the silicone elastomer (D) is blended, dewiness at the time of application may be lost.

The cosmetic of the present invention further contains (E) a silicone-based surfactant. As the silicone-based surfactant used in the present invention, polyether-modified silicones obtained by introducing polyoxyalkylene structures into silicone skeletons are preferably used. For example, crosslinked products obtained by crosslinking silicone chains by polyoxyalkylene chains, and side chain type polyether-modified silicones obtained by introducing polyoxyalkylene groups as side chains into silicone chains are preferred.

Specific examples of the silicone-based surfactant include polyether-modified silicones such as PEG-10 dimethicone, PEG-9 polydimethylsiloxyethyl dimethicone, lauryl PEG-9 polydimethylsiloxyethyl dimethicone, and lauryl PEG-15 polydimethylsiloxyethyl dimethicone, and crosslinked polyether-modified silicones such as (dimethicone/(PEG10/15)) crosspolymers and (dimethicone/polyglycerin-3) crosspolymers. One of these can be blended, or two or more of these can be mixed and blended.

Crosslinked polyether-modified silicones such as (dimethicone/(PEG-10/15)) crosspolymers can act not only as the silicone-based surfactant (E) but also as the above silicone elastomer (D), and those having a function as a surfactant are herein classified into the silicone-based surfactant (E) and distinguished.

In addition, the silicone-based surfactant (E) may also be blended in a state of a silicone gel comprising the surfactant and a solvent, and commercially available silicone gels can be used. For example, KSG-210, KSG-710, and KSG-360Z (manufactured by Shin-Etsu Chemical Co., Ltd.) are illustrated. It is needless to say that the fluid volatile oils contained in these silicone gels as solvents are also classified into the above component (A), and the amount of the fluid volatile oil blended is added to the amount of the component (A) blended.

The amount of the silicone-based surfactant (E) blended in the cosmetic of the present invention is 0.5 to 5% by mass. When the amount blended is less than 0.05% by mass, the emulsification stability decreases. When more than 20% by mass of the silicone-based surfactant (E) is blended, stickiness may be caused to lost dewiness.

In addition to the above essential components (A) to (E), the cosmetic of the present invention contains other components that can be blended into the stick cosmetic, as long as they do not impair the effects of the invention.

(F) Moisturizing Agent

By blending a moisturizing agent, the moisture-retaining power of the skin after application can be improved. The moisturizing agent also contributes to the suppression of the "thinning" of the stick due to the evaporation of the blended water.

Specific examples of the moisturizing agent include glycols such as propylene glycol, dipropylene glycol, 1,3-butylene glycol, 1,4-butylene glycol, diethylene glycol, triethylene glycol, and polyethylene glycol; glycerins such as glycerin, diglycerin, and polyglycerin; sugar alcohols such as sorbitol, mannitol, maltitol, xylitol, and erythritol; and saccharides such as fructose, glucose, galactose, maltose, lactose, and trehalose.

When the moisturizing agent is blended into the cosmetic of the present invention, the amount of the moisturizing agent blended is 5 to 20% by mass. When the amount blended is less than 0.1% by mass, a sufficient moisturizing effect is less likely to be obtained, and the thinning of the stick may be likely to occur. When more than 50% by mass of the moisturizing agent is blended, stickiness may be caused to lost dewiness.

(G) Fluid nonvolatile oil

By blending a fluid nonvolatilefluid volatile oil, the hardness of the stick cosmetic can be adjusted. Further, because the fluid nonvolatile oil can cooperate with the above fluid volatile oil to dissolve makeup applied to the skin, a local makeup removal (cleansing) function is exhibited, and a mode suitable for easy fixing of makeup is thus provided.

The "fluid nonvolatile oil" herein refers to a liquid oil that has fluidity at normal temperature (25°C) and does not apply to the above volatile oil (A). The fluid nonvolatile oil can be selected from oils that can be used in cosmetics and the like. For example, one or two or more hydrocarbon oils or silicone oils of oily components having a boiling point of higher than 260°C at normal pressure or silicone oils having a viscosity of more than 10 cs can be used.

Specific examples of the fluid nonvolatile oil may include liquid oils and fats such as linseed oil, camellia oil, macadamia nut oil, corn oil, olive oil, avocado oil, sasanqua oil, castor oil, safflower oil, apricot kernel oil, cinnamon oil, grape oil, almond oil, rapeseed oil, sesame oil, sunflower oil, wheat germ oil, rice germ oil, rice bran oil, cottonseed oil, soybean oil, peanut oil, tea seed oil, evening primrose oil, yolk oil, liver oil, triglycerin, glyceryl trioctanoate, and glyceryl triisopalmitate; ester oils such as octanoates such as cetyl octanoate, isooctanoates such as glyceryl tri-2-ethylhexanoate and pentaerythritol tetra-2-ethylhexanoate, laurates such as hexyl laurate, myristates such as isopropyl myristate and octyldodecyl myristate, palmitates such as octyl palmitate, stearates such as isocetyl stearate, isostearates such as isopropyl isostearate, isopalmitates such as octyl isopalmitate, oleates such as isodecyl oleate, adipic acid diesters such as diisopropyl adipate, sebacic acid diesters such as diethyl sebacate, and ester oils such as diisostearyl malate; liquid hydrocarbon oils such as liquid paraffins and squalane; and further chain silicones such as dimethylpolysiloxane, methylphenylpolysiloxane, and methylhydrogenpolysiloxane, amino-modified silicones, carboxy-modified silicones, alkyl-modified silicones, ammonium salt-modified silicones, and fluorine-modified silicones as silicone oils.

When the fluid nonvolatile oil is blended into the cosmetic of the present invention, an amount of the fluid nonvolatile oil blended is 1 to 15% by mass. When the amount blended is less than 0.1% by mass, the cosmetic is poorly spread, and chipping or crumbling thereof may not be sufficiently suppressed. When more than 50% by mass of the fluid nonvolatile oil is blended, the cosmetic becomes sticky and moisturizing effect may be lost.

As the optional components, the following are illustrated.

(H) Powder Component

The cosmetic of the present invention may contain a variety of powder components, which can be blended into skin cosmetics. Specific examples of the powder components include inorganic powders such as talc, kaolin, mica, silica, and zeolite; organic powders such as polyamide resin powders (nylon powders), polyethylene powders, polymethyl methacrylate powders, polystyrene powders, powders of copolymer resins of styrene and acrylic acid, and cellulose powders; inorganic white pigments such as titanium dioxide and zinc oxide; inorganic red pigments such as iron oxide (red oxide); inorganic yellow pigments such as yellow iron oxide and ocher; black pigments such as black iron oxide and carbon black; inorganic green pigments such as chromium oxide, chromium hydroxide, and cobalt titanate; inorganic blue pigments such as ultramarine blue and Prussian blue; pearl pigments such as titanium oxide-coated mica, colored titanium oxide-coated mica, bismuth oxychloride, and argentine; metal powder pigments such as aluminum powders and copper powders; organic pigments such as Red No. 201, Red No. 202, Red No. 204, Red No. 205, Red No. 220, Red No. 226, Red No. 228, Red No. 405, Orange No. 203, Orange No. 204, Yellow No. 205, Yellow No. 401, and Blue No. 404; organic pigments such as zirconium, barium, or aluminum lakes such as Red No. 3, Red No. 104, Red No. 106, Red No. 227, Red No. 230, Red No. 401, Red No. 505, Orange No. 205, Yellow No. 4, Yellow No. 5, Yellow No. 202, Yellow No. 203, Green No. 3, and Blue No. 1; and natural coloring matter such as chlorophyll and β-carotene.

When the powder component is blended into the cosmetic of the present invention, an amount of the powder component blended is usually 0.00001 to 30% by mass, preferably 0.00001 to 20% by mass, and more preferably 0.00001 to 10% by mass. When the amount blended is less than 0.00001% by mass, a feeling of use unique to a powder such as a dry feeling may not be sufficiently felt, or color may not be sufficiently produced. When more than 30% by mass of the powder component is blended, a moisturizing effect and dewiness may be lost.

(I) Other Optional Components

Examples of optional components other than the above include thickening agents, ceramides, vitamins, ultraviolet absorbing agents, chelating agents, bactericides, preservatives, plant extracts, amino acids, various drugs, and lower alcohols such as ethanol.

The cosmetic of the present invention is a stick cosmetic that has the new function of skin moisture replenishment during the daytime and is suitable for portable use for fixing makeup.

The cosmetic of the present invention can be produced according to a method conventionally used for a stick cosmetic. To put it briefly, the cosmetic of the present invention can be produced by separately mixing oily components and aqueous components with heating as needed, emulsifying the aqueous phase in the oil phase, then filling a container with the obtained emulsion, and slowly cooling the emulsion.

A hardness of the cosmetic of the present invention is sufficient as a stick cosmetic. Specifically, the hardness thereof is preferably 5 to 120, more preferably 7 to 70, and further preferably 10 to 50. The hardness is specified as a value (γ) represented by the above formula (1).

The stick cosmetic of the present invention containing a larger amount of water in the internal phase than a conventional stick cosmetic is a stick applicable directly without transferring the cosmetic to hands or fingers, so that when directly applied to the skin, the emulsification state goes out and consequently, a large amount of water can be liberated to provide a well moistened feeling.

On the other hand, the blended moderate amounts of oily components and a silicone elastomer having an correcting effect on roughness of the skin, so that the stick cosmetic of the present invention is also preferred for fixing the makeup on the makeup skin treated while ago.

As a specific method for fixing makeup, the following methods can be applied.

(1) Removing an excess sebum and sweat and pressing an oil-blotting paper or a tissue (or wiping off), as needed.
(2) Applying the cosmetic of the present invention directly to a portion where the makeup has become irregular.
(3) Smoothly adapting the cosmetic of the present invention by pressing it with tissue, or the like, as needed.
(4) Applying a powder foundation or a powder over the adopted portion.

Examples

The present invention will be described in more detail below by Examples, but the present invention is not limited in any way by these Examples. Amounts blended mean "% by mass" unless otherwise specified.

Stick cosmetics were prepared according to formulations shown in the following Table 1 to Table 4, and evaluations were performed as for the following evaluation terms (1) to (11) according to the following evaluation methods and evaluation criteria. The results are shown together in the tables. Meantime, the hardness in the tables is represented by a hardness ($\gamma$) measured under the conditions described in the paragraph [0018].

• Evaluation Items

    (1) Forming a solid stick and formability therefor
    (2) No stickiness
    (3) Not glare
    (4) No irregularity as for the foundation
    (5) Well spread
    (6) No chipping or crumbling the stick
    (7) Moistened feeling on an application thereof
    (8) Soft and comfortable when applied to skin
    (9) Moisturizing effect (effect of replenishing)
    (10) Irregularity and roughness correcting effect
    (11) Thinning of the stick

• Evaluation Methods and Evaluation Criteria Regarding Evaluation Item (1)

<Evaluation Method>

The oily components and the aqueous components contained in the composition of each example were separately mixed under heating, and the aqueous phase was emulsified in the oil phase. Then, a container was filled with the obtained emulsion, and the emulsion was slowly cooled.

<Evaluation Criteria>

+: The emulsion becomes solid and formable to a stick.
-: The emulsion separates and does not become solid and form a stick

Regarding Evaluation Items (2) to (10)

<Evaluation Method>

10 expert panelists applied each test sample and provide the evaluation result according to the following criteria:

<Evaluation Criteria>

    A: At least 9 out of 10 agreed excellent.
    B: 7 to 8 out of 10 agreed excellent.
    C: 3 to 6 out of 10 agreed excellent.
    D: At most 2 out of 10 agreed excellent.

Regarding Evaluation Item (11)

<Evaluation Method>

Each sample weight right after the sample is prepared (initial value) and 4 weeks later after standing at room temperature ware tared and calculated respective weight decrease percentages.

<Evaluation Criteria>

A: The weight decrease rate is not more than 0.3% of the initial weight value.
B: The weight decrease rate is more than 0.3% but not more than 0.5% of the initial weight value.
C: The weight decrease rate is more than 0.5% but not more than 0.8% of the initial weight value.
D: The weight decrease rate is not less than 0.8% of the initial value.

[Table 1]

| | | Example 1 | Example 2 | Example 3 | Example 4 | Example 5 | Example 6 |
|---|---|---|---|---|---|---|---|
| (B) | Water | 53.7 | 54.2 | 57.2 | 57.2 | 57.2 | 52.2 |
| (F) | Glycerin | 7 | 7 | 7 | 7 | 7 | 7 |
| | 1,3-Butylene glycol | 7 | 7 | 7 | 7 | 7 | 7 |
| (E) | PEG-10 dimethicone | 1 | 1 | 1 | 1 | 1 | 1 |
| | PEG-9 polydimethylsiloxyethyl dimethicone | - | - | - | - | - | - |
| | Lauryl PEG-9 polydimethylsiloxyethyl dimethicone | - | - | - | - | - | - |
| | KSG-210 (*1) | 1.5 | 1.5 | 1.5 | 1.5 | 1.5 | 1.5 |
| | KSG-710 (*2) | - | - | - | - | - | - |
| | KSG-360Z (*3) | - | - | - | - | - | - |
| (A) | Decamethylcyclopentasiloxane | 13.5 | - | - | 3 | 3 | 13 |
| | Dimethicone (1.5 cs) | - | 10 | - | - | - | - |
| | Dimethicone (2 cs) | - | - | 10 | - | - | - |
| | Isododecane | - | - | - | 5 | - | - |
| | Isohexadecane | - | - | - | - | 5 | - |
| | Dimethicone (6 cs) | 3 | 6 | 3 | 5 | 5 | 3 |
| (G) | Dimethicone (20 cs) | - | - | - | - | - | 2 |
| | Methylphenylpolysiloxane | - | - | - | - | - | - |
| | Glyceryl tri2-ethylhexanoate | 1 | 1 | 1 | 1 | 1 | 1 |
| | Isodecyl neopentanoate | - | - | - | - | - | - |
| | Liquid paraffin | - | - | - | - | - | - |
| (C) | Myristyl myristate | 2 | 2 | 2 | 2 | 2 | 2 |
| | Paraffin | 0.4 | 0.4 | 0.4 | 0.4 | 0.4 | 0.4 |
| | Microcrystalline wax | 4.6 | 4.6 | 4.6 | 4.6 | 4.6 | 4.6 |
| | Polyethylene wax | - | - | - | - | - | - |
| | Hydrogenated jojoba oil | - | - | - | - | - | - |

EP 3 143 984 B1

(continued)

| | | Example 1 | Example 2 | Example 3 | Example 4 | Example 5 | Example 6 |
|---|---|---|---|---|---|---|---|
| (D) | KSG-18A (*4) | 4 | 4 | 4 | 4 | 4 | 4 |
| | KSG15AP (*5) | - | - | - | - | - | - |
| | Elastomer blend DC9045 (*6) | - | - | - | - | - | - |
| (I) | Dextrin palmitate | - | - | - | - | - | - |
| | Disodium edetate | 0.05 | 0.05 | 0.05 | 0.05 | 0.05 | 0.05 |
| | Sodium chloride | 1 | 1 | 1 | 1 | 1 | 1 |
| | Methylparaben | 0.1 | 0.1 | 0.1 | 0.1 | 0.1 | 0.1 |
| | Ethylparaben | 0.15 | 0.15 | 0.15 | 0.15 | 0.15 | 0.15 |
| Hardness (1.5$\phi$, 25°C) | | 28 | 35 | 32 | 37 | 34 | 31 |
| (1) Forming a solid stick and formability therefor | | + | + | + | + | + | + |
| (2) No stickiness | | A | A | A | A | A | A |
| (3) Not glare | | A | A | A | A | A | A |
| (4) No irregularity as for the foundation | | A | A | A | A | A | A |
| (5) Well spread | | A | A | A | A | A | A |
| (6) No chipping or crumbling the stick | | A | A | A | A | A | A |
| (7) Moistened feeling on an application thereof | | A | A | A | A | A | A |
| (8) Soft and comfortable when applied to skin | | A | A | A | A | A | A |
| (9) Moisturizing effect (effect of replenishing) | | A | A | A | A | A | A |
| (10) Irregularity and roughness correcting effect | | A | A | A | A | A | A |
| (11) Thinning of the stick | | A | A | A | A | A | A |

[Table 2]

| | | Example 7 | Example 8 | Example 9 | Example 10 | Example 11 | Example 12 |
|---|---|---|---|---|---|---|---|
| (B) | Water | 57.65 | 27.2 | 53.7 | 53.7 | 53.7 | 55.7 |
| (F) | Glycerin | 7 | 7 | 7 | 7 | 7 | 7 |
| | 1,3-Butylene glycol | 7 | 7 | 7 | 7 | 7 | 7 |
| (E) | PEG-10 dimethicone | 1 | 1 | 1 | 1 | 1 | 1 |
| | PEG-9 polydimethylsiloxyethyl dimethicone | - | - | - | - | - | - |
| | Lauryl PEG-9 polydimethylsiloxyethyl dimethicone | - | - | - | - | - | - |
| | KSG-210 (*1) | 1.5 | 1.5 | 1.5 | 1.5 | 1.5 | 1.5 |
| | KSG-710 (*2) | - | - | - | - | - | - |
| | KSG-360Z (*3) | - | - | - | - | - | - |
| (A) | Decamethylcyclopentasiloxane | - | - | 13.5 | 13.5 | 13.5 | 13.5 |
| | Dimethicone (1.5 cs) | - | - | - | - | - | - |
| | Dimethicone (2 cs) | - | - | - | - | - | - |
| | Isododecane | 13.5 | 25 | - | - | - | - |
| | Isohexadecane | - | - | - | - | - | - |
| | Dimethicone (6 cs) | 3 | - | 3 | 3 | 3 | 3 |
| (G) | Dimethicone (20 cs) | - | - | - | - | - | - |
| | Methylphenylpolysiloxane | - | - | - | - | - | - |
| | Glyceryl tri2-ethylhexanoate | 1 | 1 | 1 | 1 | 1 | 1 |
| | Isodecyl neopentanoate | - | - | - | - | - | - |
| | Liquid paraffin | - | - | - | - | - | - |
| (C) | Myristyl myristate | - | - | 2 | 2 | 2 | - |
| | Paraffin | 0.004 | 2 | - | - | - | - |
| | Microcrystalline wax | 0.046 | 23 | 5 | 0.75 | 3.75 | - |
| | Polyethylene wax | - | - | - | 4.25 | 1.25 | - |
| | Hydrogenated jojoba oil | - | - | - | - | - | 5 |

(continued)

| | | Example 7 | Example 8 | Example 9 | Example 10 | Example 11 | Example 12 |
|---|---|---|---|---|---|---|---|
| (D) | KSG-18A (*4) | 4 | 4 | 4 | 4 | 4 | 4 |
| | KSG15AP (*5) | - | - | - | - | - | - |
| | Elastomer blend DC9045 (*6) | - | - | - | - | - | - |
| (I) | Dextrin palmitate | 3 | - | - | - | - | - |
| | Disodium edetate | 0.05 | 0.05 | 0.05 | 0.05 | 0.05 | 0.05 |
| | Sodium chloride | 1 | 1 | 1 | 1 | 1 | 1 |
| | Methylparaben | 0.1 | 0.1 | 0.1 | 0.1 | 0.1 | 0.1 |
| | Ethylparaben | 0.15 | 0.15 | 0.15 | 0.15 | 0.15 | 0.15 |
| Hardness (1.5$\phi$, 25°C) | | 7 | 102 | 10 | 15 | 14 | 45 |
| (1) Forming a solid stick and formability therefor | | + | + | + | + | + | + |
| (2) No stickiness | | B | B | A | A | A | B |
| (3) Not glare | | B | B | A | A | A | A |
| (4) No irregularity as for the foundation | | B | B | A | A | A | A |
| (5) Well spread | | B | B | A | A | A | B |
| (6) No chipping or crumbling the stick | | B | B | A | A | A | B |
| (7) Moistened feeling on an application thereof | | B | B | A | A | A | B |
| (8) Soft and comfortable when applied to skin | | B | B | A | A | A | A |
| (9) Moisturizing effect (effect of replenishing) | | A | A | A | A | A | A |
| (10) Irregularity and roughness correcting effect | | A | A | A | A | A | A |
| (11) Thinning of the stick | | A | A | A | A | A | A |

[Table 3]

| | | | Example 13 | Example 14 | Example 15 | Example 16 | Example 17 |
|---|---|---|---|---|---|---|---|
| (B) | | Water | 53.7 | 53.7 | 57.65 | 57.7 | 51.7 |
| (F) | | Glycerin | 7 | 7 | 7 | 7 | 7 |
| | | 1,3-Butylene glycol | 7 | 7 | 7 | 3 | 7 |
| (E) | | PEG-10 dimethicone | 1 | 1 | 1 | - | - |
| | | PEG-9 polydimethylsiloxyethyl dimethicone | - | - | - | 1 | - |
| | | Lauryl PEG-9 polydimethylsiloxyethyl dimethicone | - | - | - | - | 1 |
| | | KSG-210 (*1) | 1.5 | 1.5 | 1.5 | - | - |
| | | KSG-710 (*2) | - | - | - | - | 1.5 |
| | | KSG-360Z (*3) | - | - | - | 1.5 | - |
| (A) | | Decamethylcyclopentasiloxane | 13.5 | 13.5 | 13.5 | 13.5 | 13.5 |
| | | Dimethicone (1.5 cs) | - | - | - | - | - |
| | | Dimethicone (2 cs) | - | - | - | - | - |
| | | Isododecane | - | - | - | - | - |
| | | Isohexadecane | - | - | - | - | - |
| | | Dimethicone (6 cs) | 3 | 3 | 3 | 3 | 3 |
| (G) | | Dimethicone (20 cs) | - | - | - | - | 2 |
| | | Methylphenylpolysiloxane | - | - | - | - | - |
| | | Glyceryl tri2-ethylhexanoate | 1 | 1 | 1 | 1 | 1 |
| | | Isodecyl neopentanoate | - | - | - | - | - |
| | | Liquid paraffin | - | - | - | - | - |
| (C) | | Myristyl myristate | 2 | 2 | 2 | 2 | 2 |
| | | Paraffin | 0.4 | 0.4 | 0.4 | 0.4 | 0.4 |
| | | Microcrystalline wax | 4.6 | 4.6 | 4.6 | 4.6 | 4.6 |
| | | Polyethylene wax | - | - | - | - | - |
| | | Hydrogenated jojoba oil | - | - | - | - | - |
| (D) | | KSG-18A (*4) | - | - | 0.05 | 4 | 4 |
| | | KSG15AP (*5) | 4 | - | - | - | - |
| | | Elastomer blend DC9045 (*6) | - | 4 | - | - | - |
| (I) | | Dextrin palmitate | - | - | - | - | - |
| | | Disodium edetate | 0.05 | 0.05 | 0.05 | 0.05 | 0.05 |
| | | Sodium chloride | 1 | 1 | 1 | 1 | 1 |
| | | Methylparaben | 0.1 | 0.1 | 0.1 | 0.1 | 0.1 |
| | | Ethylparaben | 0.15 | 0.15 | 0.15 | 0.15 | 0.15 |
| Hardness (1.5$\phi$, 25°C) | | | 27 | 22 | 28 | 28 | 23 |

(continued)

|  | Example 13 | Example 14 | Example 15 | Example 16 | Example 17 |
|---|---|---|---|---|---|
| (1) Forming a solid stick and formability therefor | + | + | + | + | + |
| (2) No stickiness | A | A | A | A | A |
| (3) Not glare | A | A | A | A | A |
| (4) No irregularity as for the foundation | A | A | A | A | A |
| (5) Well spread | A | A | A | A | A |
| (6) No chipping or crumbling the stick | A | A | A | A | A |
| (7) Moistened feeling on an application thereof | A | A | A | A | A |
| (8) Soft and comfortable when applied to skin | A | A | A | A | A |
| (9) Moisturizing effect (effect of replenishing) | A | A | A | A | A |
| (10) Irregularity and roughness correcting effect | A | A | C | A | A |
| (11) Thinning of the stick | A | A | B | A | A |

[Table 4]

| | | Comparative Example 1 | Comparative Example 2 | Comparative Example 3 | Comparative Example 4 | Comparative Example 5 |
|---|---|---|---|---|---|---|
| (B) | Water | 51.195 | 10.7 | 4.7 | 61.65 | 60.7 |
| (F) | Glycerin | 7 | 5 | 5 | 7 | 7 |
| | 1,3-Butylene glycol | 7 | 3 | 3 | 7 | 7 |
| | PEG-10 dimethicone | 1 | 1 | 1 | 1 | - |
| | PEG-9 polydimethylsiloxyethyl dimethicone | - | - | - | - | - |
| (E) | KSG-310 (*7) | 1.5 | - | - | - | - |
| | KSG-210 (*1) | - | 1.5 | 1.5 | 1.5 | - |
| | KSG-710 (*2) | - | - | - | - | - |
| | KSG-360Z (*3) | - | - | - | - | - |
| (A) | Decamethylcyclopentasiloxane | 0.005 | 51 | 45 | - | 8 |
| | Dimethicone (1.5 cs) | - | - | - | - | - |
| | Dimethicone (2 cs) | - | - | - | - | - |
| | Isododecane | - | - | - | 13.5 | - |
| | Isohexadecane | - | - | - | - | - |
| | Dimethicone (6 cs) | - | - | - | 3 | 3 |
| | Dimethicone (20 cs) | 5 | - | 10 | - | - |
| | Methylphenylpolysiloxane | 10 | - | - | - | - |
| (G) | Glyceryl tri2-ethylhexanoate | 5 | 3 | 5 | 1 | 1 |
| | Isodecyl neopentanoate | - | - | - | - | - |
| | Liquid paraffin | - | - | - | - | 5 |

(continued)

| | | Comparative Example 1 | Comparative Example 2 | Comparative Example 3 | Comparative Example 4 | Comparative Example 5 |
|---|---|---|---|---|---|---|
| (C) | Myristyl myristate | 2 | 3.5 | 3.5 | - | 2 |
| | Paraffin | 0.4 | 0.8 | 0.8 | 0.004 | 0.4 |
| | Microcrystalline wax | 4.6 | 9.2 | 9.2 | 0.046 | 4.6 |
| | Polyethylene wax | - | - | - | - | - |
| | Hydrogenated jojoba oil | - | - | - | - | - |
| (D) | KSG-18A (*4) | 4 | 10 | 10 | - | - |
| | KSG15AP (*5) | - | - | - | - | - |
| | Elastomer blend DC9045 (*6) | - | - | - | - | - |
| (I) | Dextrin palmitate | - | - | - | 3 | - |
| | Disodium edetate | 0.05 | 0.05 | 0.05 | 0.05 | 0.05 |
| | Sodium chloride | 1 | 1 | 1 | 1 | 1 |
| | Methylparaben | 0.1 | 0.1 | 0.1 | 0.1 | 0.1 |
| | Ethylparaben | 0.15 | 0.15 | 0.15 | 0.15 | 0.15 |
| Hardness (1.5$\phi$, 25°C) | | 35 | 50 | 46 | - | 1 |
| (1) Forming a solid stick and formability therefor | | + | + | + | - | - |
| (2) No stickiness | | D | D | D | - | - |
| (3) Not glare | | D | D | D | - | - |
| (4) No irregularity as for the foundation | | D | D | D | - | - |
| (5) Well spread | | B | C | C | - | - |
| (6) No chipping or crumbling the stick | | B | D | D | - | - |
| (7) Moistened feeling on an application thereof | | C | C | D | - | - |
| (8) Soft and comfortable when applied to skin | | B | C | C | - | - |
| (9) Moisturizing effect (effect of replenishing) | | A | B | B | - | - |
| (10) Irregularity and roughness correcting effect | | A | A | A | - | - |

|  | | Comparative Example 1 | Comparative Example 2 | Comparative Example 3 | Comparative Example 4 | Comparative Example 5 |
|---|---|---|---|---|---|---|
| (11) Thinning of the stick | | A | A | A | - | - |

[Table 5]

| | | | Example 18 | Example 19 |
|---|---|---|---|---|
| (B) | | Water | 64.7 | 12.7 |
| (F) | | Glycerin | 3 | 55 |
| | | 1,3-Butylene glycol | - | - |
| (E) | | PEG-10 dimethicone | 1 | 1 |
| | | PEG-9 polydimethylsiloxyethyl dimethicone | - | - |
| | | Lauryl PEG-9 polydimethylsiloxyethyl dimethicone | - | - |
| | | KSG-210 (*1) | 1.5 | 1.5 |
| | | KSG-710 (*2) | - | - |
| | | KSG-360Z (*3) | - | - |
| (A) | | Decamethylcyclopentasiloxane | 13.5 | 13.5 |
| | | Dimethicone (1.5 cs) | - | - |
| | | Dimethicone (2 cs) | - | - |
| | | Isododecane | - | - |
| | | Isohexadecane | - | - |
| | | Dimethicone (6 cs) | 3 | 3 |
| (G) | | Dimethicone (20 cs) | - | - |
| | | Methylphenylpolysiloxane | - | - |
| | | Glyceryl tri2-ethylhexanoate | 1 | 1 |
| | | Isodecyl neopentanoate | - | - |
| | | Liquid paraffin | - | - |
| (C) | | Myristyl myristate | 2 | 2 |
| | | Paraffin | 0.4 | 0.4 |
| | | Microcrystalline wax | 4.6 | 4.6 |
| | | Polyethylene wax | - | - |
| | | Hydrogenated jojoba oil | - | - |
| (D) | | KSG-18A (*4) | 4 | 4 |
| | | KSG15AP (*5) | - | - |
| | | Elastomer blend DC9045 (*6) | - | - |
| (I) | | Dextrin palmitate | - | - |
| | | Disodium edetate | 0.05 | 0.05 |
| | | Sodium chloride | 1 | 1 |
| | | Methylparaben | 0.1 | 0.1 |
| | | Ethylparaben | 0.15 | 0.15 |
| Hardness (1.5ϕ, 25°C) | | | 26 | 26 |
| (1) Forming a solid stick and formability therefor | | | + | + |
| (2) No stickiness | | | A | B |
| (3) Not glare | | | A | B |

(continued)

|  |  | Example 18 | Example 19 |
|---|---|---|---|
| (4) No irregularity as for the foundation | | A | B |
| (5) Well spread | | A | B |
| (6) No chipping or crumbling the stick | | A | B |
| (7) Moistened feeling on an application thereof | | A | B |
| (8) Soft and comfortable when applied to skin | | A | A |
| (9) Moisturizing effect (effect of replenishing) | | B | A |
| (10) Irregularity and roughness correcting effect | | A | A |
| (11) Thinning of the stick | | B | A |

[Table 6]

Footnotes to Tables:

*1: 20 to 30% of a (dimethicone/(PEG-10/15)) crosspolymer/70 to 80% of dimethicone (6 cs) (manufactured by Shin-Etsu Chemical Co., Ltd.)
*2: 20 to 30% of a (dimethicone/polyglycerin-3) crosspolymer/70 to 80% of dimethicone (6 cs) (manufactured by Shin-Etsu Chemical Co., Ltd.)
*3: 30 to 40% of a (PEG-15/lauryl polydimethylsiloxyethyl dimethicone) crosspolymer/60 to 70% of dimethicone (6 cs) (manufactured by Shin-Etsu Chemical Co., Ltd.)
*4: 10 to 20% of a dimethicone/phenyl vinyl dimethicone) crosspolymer/80 to 90% of diphenyl siloxyphenyl trimethicone (manufactured by Shin-Etsu Chemical Co., Ltd.)
*5: 4 to 10% of a dimethicone/vinyl dimethicone crosspolymer/90 to 96% of decamethylcyclopentasiloxane (manufactured by Shin-Etsu Chemical Co., Ltd.)
*6: 12.5% of a dimethicone crosspolymer/87.5% of decamethylcyclopentasiloxane (manufactured by Dow Corning Toray Co., Ltd.)
*7: 25 to 35% of a (PEG-15/lauryl dimethicone) crosspolymer/65 to 75% of a mineral oil (manufactured by Shin-Etsu Chemical Co., Ltd.)

As shown in Table 4, Comparative Example 1 having less than 0.01% by mass of the fluid volatile oil (A); on the contrary, Comparative Example 2 having more than 50% by mass of the fluid volatile oil (A) ; Comparative Example 3 having less than 10% by mass of the water (B); and Comparative Example 4 having no silicone elastomer (D); and further Comparative Example 5 having no silicone-based surfactant (E); have failed to meet a few expected requirements. On the other hand, Examples 1 to 6, 9 to 14, 16 to 18 (Tables 1 to 3 and Table 5) could meet all expected requirements for the commercial products, so that examples 1 to 6, 9 to 14, 16 to 18 of the present invention have satisfactorily excellent properties as to all measurement item.
Non-limiting formulation examples of the cosmetic of the present invention are shown below. These cosmetics also had excellent properties similar to those of the above Examples 1 to 6, 9 to 14, 16 to 18.

<Formulation Example 1: Stick cosmetic-serum (Essence)>

| Component | Amount blended (% by mass) |
|---|---|
| (1) water | the remainder |
| (2) glycerin | 7 |
| (3) 1,3-butylene glycol | 2 |
| (4) dipropylene glycol | 9 |
| (5) trehalose | 2 |
| (6) xylitol | 0.5 |
| (7) lauryldimethylaminoacetic acid betaine | 0.5 |

(continued)

| Component | Amount blended (% by mass) |
|---|---|
| (8) PEG-9 polydimethylsiloxyethyl dimethicone | 1.2 |
| (9) KSG-210 (*1) | 1.5 |
| (10) decamethylcyclopentasiloxane | 10 |
| (11) isostearic acid | 0.5 |
| (12) dimethicone (6 cs) | 4 |
| (13) pentaerythritol tetra2-ethylhexanoate | 2 |
| (14) myristyl myristate | 2 |
| (15) paraffin | 0.32 |
| (16) microcrystalline wax | 3.68 |
| (17) KSG-18A (*4) | 4 |
| (18) polyquaternium-51 | 1 |
| (19) dipotassium glycyrrhizinate | 0.01 |
| (20) green tea extract | 0.01 |
| (21) trisodium edetate | 0.05 |
| (22) sodium chloride | 1 |
| (23) methylparaben | 0.1 |
| (24) ethylparaben | 0.15 |

<Production Method>

For the above formulation, the oil phase components (8) to (17) and (24) were heated and mixed at 90°C, and while the mixture was stirred by a homomixer, a mixture of the aqueous phase components (1) to (7) and (18) to (23) heated to 85°C was gradually added. A stick container was filled with the mixture, and then the mixture was slowly cooled.

<Formulation Example 2: Stick skin-whitening cosmetic-serum (Essence)>

| Component | Amount blended (% by mass) |
|---|---|
| (1) water | the remainder |
| (2) glycerin | 6 |
| (3) 1,3-butylene glycol | 8 |
| (4) dipropylene glycol | 9 |
| (5) polyethylene glycol 6000 | 1 |
| (6) PEG-9 polydimethylsiloxyethyl dimethicone | 1.2 |
| (7) KSG-210 (*1) | 1.5 |
| (8) decamethylcyclopentasiloxane | 7 |
| (9) dimethicone (6 cs) | 5 |
| (10) glyceryl tri2-ethylhexanoate | 1 |
| (11) myristyl myristate | 2 |
| (12) paraffin | 0.4 |
| (13) microcrystalline wax | 4.6 |
| (14) elastomer blend DC9045 (*6) | 5 |
| (15) octyl methoxycinnamate | 0.05 |
| (16) tranexamic acid | 2 |
| (17) potassium 4-methoxysalicylate | 1 |
| (18) acetic acid dl-$\alpha$-tocopherol | 0.05 |
| (19) sweet tea extract | 0.01 |
| (20) yeast extract | 0.01 |
| (21) Mortierella oil | 0.01 |
| (22) polyoxyethylene (14) polyoxypropylene (7) dimethyl ether | 1 |

(continued)

| Component | Amount blended (% by mass) |
|---|---|
| (23) disodium edetate | 0.05 |
| (24) sodium chloride | 1 |
| (25) phenoxyethanol | 0.05 |
| (26) perfume | 0.01 |

<Production Method>

For the above formulation, the oil phase components (6) to (15), (18), (21), and (26) were heated and mixed at 90°C, and while the mixture was stirred by a homomixer, a mixture of the aqueous phase components (1) to (5), (16) to (17), (19) to (20), and (22) to (25) heated to 85°C was gradually added. A stick container was filled with the mixture, and then the mixture was slowly cooled.

<Formulation Example 3: Stick moisturizing balm>

| Component | Amount blended (% by mass) |
|---|---|
| (1) water | the remainder |
| (2) glycerin | 13 |
| (3) 1,3-butylene glycol | 8 |
| (4) potassium hydroxide | 0.1 |
| (5) PEG-9 polydimethylsiloxyethyl dimethicone | 1.2 |
| (6) KSG-210 (*1) | 1.5 |
| (7) isododecane | 3.5 |
| (8) diphenyl siloxyphenyl trimethicone | 6.5 |
| (9) dimethicone (2 cs) | 8 |
| (10) pentaerythritol tetra2-ethylhexanoate | 2 |
| (11) Vaseline | 2 |
| (12) microcrystalline wax | 0.75 |
| (13) polyethylene wax | 4.25 |
| (14) KSG-18A (*4) | 4 |
| (15) D-methionine | 1 |
| (16) zinc sulfocarbolate | 0.2 |
| (17) fermented soybean extract | 0.1 |
| (18) lavender oil | 0.001 |
| (19) β-carotene | 0.00005 |
| (20) disodium edetate | 0.05 |
| (21) sodium chloride | 1 |
| (22) methylparaben | 0.1 |
| (23) ethylparaben | 0.15 |

<Production Method>

For the above formulation, the oil phase components (5) to (14), (18), (19), and (23) were heated and mixed at 90°C, and while the mixture was stirred by a homomixer, a mixture of the aqueous phase components (1) to (4), (15) to (17), and (20) to (22) heated to 85°C was gradually added. A stick container was filled with the mixture, and then the mixture was slowly cooled.

**Claims**

1. A stick cosmetic for moisture replenishment comprising:

   (A) 5 to 20% by mass of a fluid volatile oil;
   (B) 45% by mass or more of water;
   (C) 3 to 10% by mass of a wax capable of gelating the fluid volatile oil;
   (D) 0.1 to 2% by mass of one or two or more silicone elastomer selected from the group consisting of (dimethicone/ phenylvinyldimethicone)crosspolymers, (dimethicone/vinyldimethicone/methicone) crosspolymers, (dimethicone/vinyldimethicone) crosspolymers, dimethicone crosspolymers, and (diphenyldimethicone/vinyldiphenyldimethicone/ silsesquioxane) crosspolymers;
   (E) 0.5 to 5% by mass of a silicone surfactant selected from polyether modified silicones;
   (F) 5 to 20% by mass of a moisturizing agent; and
   (G) 1 to 15% by mass of a fluid nonvolatile oil.

2. The cosmetic according to claim 1, having a hardness of 5 to 120 as measured by a rheometer, according to the description.

**Patentansprüche**

1. Stift-Kosmetikum zur Feuchtigkeitsnachfüllung, umfassend:

   (A) 5 bis 20 Masse-% eines flüssigen flüchtigen Öls;
   (B) 45 Masse -% oder mehr Wasser;
   (C) 3 bis 10 Masse-% eines Wachses, welches das flüssige flüchtige Öl gelieren kann;
   (D) 0,1 bis 2 Masse-% eines oder zweier oder mehrerer Silikonelastomere, ausgewählt aus der Gruppe bestehend aus Dimethicon/Phenylvinyldimethicon-Kreuzpolymeren, Dimethicon/Vinyldimethicon/Methicon-Kreuzpolymeren, Dimethicon/Vinyldimethicon-Kreuzpolymeren, Dimethicon-Kreuzpolymeren und Diphenyldimethicon/Vinyldiphenyldimethicon/Silsesquioxan-Kreuzpolymeren;
   (E) 0,5 bis 5 Masse-% eines Silikontensids, ausgewählt aus Polyether-modifizierten Silikonen;
   (F) 5 bis 20 Masse-% eines Feuchtigkeitsspenders; und
   (G) 1 bis 15 Masse-% eines flüssigen nicht-flüchtigen Öls.

2. Kosmetikum nach Anspruch 1 mit einer Härte von 5 bis 120, gemessen mit einem Rheometer, gemäß der Beschreibung.

**Revendications**

1. Cosmétique sous forme de bâton pour la reconstitution de l'humidité, comprenant :

   (A) 5 à 20 % en masse d'une huile volatile fluide ;
   (B) 45 % en masse ou plus d'eau ;
   (C) 3 à 10 % en masse d'une cire capable de gélifier l'huile volatile fluide ;
   (D) 0,1 à 2 % en masse d'un ou deux élastomères de silicone, ou davantage, choisis dans le groupe consistant en polymères réticulés diméthicone/phénylvinyldiméthicone, polymères réticulés diméthicone/vinyldiméthicone/méthicone, polymères réticulés diméthicone/vinyldiméthicone, polymères réticulés diméthicone, et polymères réticulés diphényldiméthicone/vinyldiphényldiméthicone /silsesquioxane ;
   (E) 0,5 à 5 % en masse d'un tensio-actif silicone choisi parmi les silicones modifiés par polyéther ;
   (F) 5 à 20 % en masse d'un agent hydratant ; et
   (G) 1 à 15 % en masse d'une huile non volatile fluide.

2. Cosmétique selon la revendication 1, ayant une dureté de 5 à 120 telle que mesurée par un rhéomètre, conformément à la description.

**EP 3 143 984 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2002053425 A
- JP 2010285349 A
- JP 2013139403 A
- JP 2003342163 A